(19) Europäisches Patentamt
European Patent Office
Office européen des brevets

(11) EP 0 931 164 B1

(12) EUROPEAN PATENT SPECIFICATION

(45) Date of publication and mention of the grant of the patent:
**26.03.2003 Bulletin 2003/13**

(21) Application number: **97934337.3**

(22) Date of filing: **23.07.1997**

(51) Int Cl.[7]: **C12Q 1/60**

(86) International application number:
**PCT/US97/13321**

(87) International publication number:
**WO 98/003675 (29.01.1998 Gazette 1998/04)**

(54) **CHOLESTEROL SEPARATION AND FLUORESCENT ANALYSIS**

AUFTRENNEN VON CHOLESTERIN UND FLUORESZENZANALYSE

SEPARATION DE CHOLESTEROL ET ANALYSE DE CELUI-CI PAR FLUORESCENCE

(84) Designated Contracting States:
**DE FR GB IT**

(30) Priority: **24.07.1996 US 22354 P**

(43) Date of publication of application:
**28.07.1999 Bulletin 1999/30**

(73) Proprietor: **HELENA LABORATORIES CORPORATION**
**Beaumont Texas 77704-0752 (US)**

(72) Inventors:
- **HICKS, Debra, Linn**
  **Orange, TX 77630 (US)**
- **MERCHANT, Mark, Edwin**
  **Nederland, TX 77627 (US)**
- **GUADAGNO, Philip, Angelo**
  **Vidor, TX 77662 (US)**
- **ROBINSON, Suzan, Sha**
  **Silsbee, TX 77656 (US)**
- **MILLICAN, Stacey, Eloise**
  **Beaumont, TX 77706 (US)**
- **NAKAZATO, Tokiya**
  **Urawa-shi, Saitama 336 (JP)**

(74) Representative: **W.P. Thompson & Co.**
**Coopers Building,**
**Church Street**
**Liverpool L1 3AB (GB)**

(56) References cited:
**EP-A- 0 183 381**
**WO-A-82/00833**
**DE-A- 3 640 349**

- **DATABASE WPI Section Ch, Week 8404 Derwent Publications Ltd., London, GB; Class B04, AN 84-020095 XP002044526 & JP 58 210 000 A (NIPPON CHEMIPHAR CO) , 7 December 1983**
- **DATABASE WPI Section Ch, Week 8327 Derwent Publications Ltd., London, GB; Class B04, AN 83-702559 XP002044527 & JP 58 089 183 A (AMANO PHARM KK) , 27 May 1983**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 931 164 B1

Printed by Jouve, 75001 PARIS (FR)

## Description

### Field of the Invention

The present invention relates to reagents and methods for the quantitative determination of cholesterol and cholesterol esters in serum lipoproteins. It has particular application in the medical and laboratory diagnostic fields where it is necessary to perform testing and analysis of biological and chemical substances.

### Background of the Invention

The relationship of HDL Cholesterol to coronary heart disease was reported by D.P. Barr et al., in an article entitled: "Protein-lipid Relationships in Human Plasma, Am. J. Med., 11:480-493 (1951) and by G.J. Miller and N.E. Miller, in an article entitled: "Plasma-High Density-Lipoproteins Concentration and Development of Ischemic Heart Disease," Lancet, 1:16-19 (1976). The work of W.P. Castelli et al., as reported in "HDL Cholesterol and Other Lipids in Coronary Heart Disease - The Cooperative Lipoprotein Phenotyping Study," Circulation, 55(5):767-772 (1977) focused attention on HDL cholesterol assessment as the definitive laboratory test in determining the risk of coronary heart disease.[1] The cholesterol content of the lipoprotein fractions has been determined by ultracentrifugation, selective precipitation, and electrophoresis on several media.[2]

Clinical laboratory measurement of the serum lipoproteins is primarily due to their predictive association with risk of coronary heart disease. Current practice guiding laboratory measurement of total serum cholesterol, triglycerides, HDL cholesterol and LDL cholesterol is derived from recommendations of expert panels convened by the National Cholesterol Education Program (NCEP). The expert panels considered epidemiological, clinical, and intervention studies in developing the recommendations for treatment decision cutpoints and recommended workup sequences for adults and children.

The clinical recommendations from NCEP panels direct clinical laboratories to perform measurements of total, HDL and LDL, cholesterol and triglycerides. The triglycerides are primarily associated with chylomicrons, very low density (VLDL) and intermediate density (IDL) lipoproteins thought to be atherogenic, but the association of triglycerides with risk of coronary heart disease in epidemiological studies is ambiguous. LDL, as the validated atherogenic lipoprotein based on its cholesterol content, is the primary basis for treatment decisions in the NCEP clinical guidelines.[3] The major protein component of LDL is apolipoprotein B100 (apoB) which has been measured previously by immunoassay. The common research method for accurate LDL cholesterol quantitation and the basis for the reference method is designated beta-quantification, beta referring to the electrophoretic term for LDL. The beta-quantification technique involves a combination of ultracentrifugation and chemical precipitation.[4] The beta-quantification method gives a so-called "broad cut" LDL which includes the Lp(a) lipoprotein,[5] often referred to "lipoprotein little a". The National Cholesterol Education Program Lipoprotein Measurement Working Group (NCEP panel) in its "Recommendations for measurement of low density lipoprotein cholesterol" (NIH Publication In Press) concluded that alternative methods are needed for routine diagnostic use, preferably ones which directly separate LDL for cholesterol quantitation. One such direct method involves electrophoresis. Electrophoretic methods, reviewed in L.A. Lewis and J.J. Opplt, CRC Handbook of Electrophoresis, Vols. 1 and 2, Boca Raton, CRC Press, Inc. (1980), have a long history of use in qualitative and quantitative analysis of lipoproteins. Electrophoresis not only allows separation and quantitation of major lipoprotein classes, but, in addition, provides a visual display useful in detecting unusual or variant patterns. Agarose has been the preferred media for separation of whole lipoproteins, providing a clear background and convenience.[6] Early electrophoretic methods were, in general, considered useful for qualitative analysis but less than desirable for lipoprotein

[1] Other works that discuss HDL cholesterol assessment as the definitive laboratory test in determining the risk of coronary heart disease include W.B. Kannel et al., "Serum Cholesterol, Lipoproteins, and the Risk of Coronary Heart Disease," Ann. Inter. Med. 74(1):1-12 (1971); T. Gordon et al., "High Density Lipoprotein As a Protective Factor Against Coronary Heart Disease," The Framingham Study, Am. J. Med., 62:707-714(1977); and R.S. Galen, "HDL Cholesterol, How Good a Risk Factor," Diag. Med. 39-58, Nov/Dec (1979).

[2] O.F. Delalla, et al., "Ultracentrifugal Analysis of Serum Lipoprotein," Methods of Biochemical Analysis, Vol. 1. 459-478 (1954); M. Burstein et al., "Precipitation of chylomicrons and very low density lipoproteins from human serum with sodium lauryl sulfate," Life Science, 11:177-184 (1972); and S.A. Cobb et al., "Enzymic Determination of Cholesterol in Serum Lipoproteins Separated by Electrophoresis," Clin. Chem., 24(7):1116-1120 (1978), respectively.

[3] National Cholesterol Education Program, Second report of the expert panel on detection, evaluation and treatment of high blood cholesterol in adults (Adult Treatment Panel II), NCEP (1993).

[4] U.S. Department of Health and Human Services, "Lipid Research Clinics Program," Manual of Laboratory Operations, Second Edition, NIH Publication (1983); and J.D. Belcher et al., "Measurement of low density lipoprotein cholesterol concentration," Methods for Clinical Laboratory Measurement of Lipid and Lipoprotein Risk Factors, Washington D.C, AACC Press, 75-86 (1991).

[5] G. Utermann, "The mysteries of lipoprotein(a)," Science, 246:904-910 (1989); and J. Loscaizo, "Lipoprotein(a): a unique risk factor for atherothrombotic disease," Arteriosclerosis 10:672-679 (1990).

[6] R.P. Nobe, "Electrophoretic separation of plasma lipoproteins in agarose gel," J. Lipid Res. 9:693 (1968); F.T. Lindgren et al., "A comparison of simplified methods for lipoprotein quantitation using the analytic ultracentrifuge as a standard," Lipids, 12:278 (1977); D. Conlon et al., "Quantitative determination of high-density lipoprotein cholesterol by agarose gel electrophoresis updated," Clin. Chem., 24:227(1979); and N.M. Papadopoulos, "Hyperlipoproteinemia phenotype determination by agarose gel electrophoresis updated," Clin. Chem., 24:227-229 (1978).

quantitation because of poor precision and large systematic biases compared to other methods as reported in an article by G.R. Warnick et al., "Lipoprotein quantification: An electrophoretic method compared with the lipid research clinics method," Clin. Chem, 28:2116-20 (1982).

EP-0183 381 discloses a method for the determination of Cholesterol which comprises incubating a sample, a Cholesterol Dehydrogenase, and NAD or NADP in the presence of a surfactant and then measuring the resulting detectable change kinetically; JP-58089 183 discloses culturing a micro-organism in a medium containing cholesterol under aerobic conditions and separating NAD(P)- dependent Cholesterol Dehydrogenase from the cultured cells and culture liquid; JP-58210 000 discloses fractionating blood serum to obtain lipoprotein cholesterol and treating with a dying reagent; WO-82/00833 discloses a method for determining total cholesterol; and DE-3640349 discloses the determination of cholesterol distribution and protein fractions after electrophoretics separation.

**Summary of the Invention**

The present invention is for use in the quantitative determination of cholesterol and cholesterol esters in the lipoproteins of serum following electrophoresis. The invention is intended to be used for the assessment of the cholesterol content of the high density lipoproteins (HDL), low density lipoproteins (LDL), and very low density lipoproteins (VLDL). The methods and reagents of the invention provide for inexpensive, quick and efficient fluorescent analysis of the cholesterol content of electrophoresed lipoprotein bands.

The present system and invention separates the major lipoprotein classes using electrophoresis. The alpha band which migrates the farthest toward the anode corresponds to HDL. The next band, pre-beta, corresponds to VLDL, and the slowest moving beta band corresponds approximately to LDL. If a band appears between alpha and the origin, with fast pre-beta mobility, it corresponds to LDL and should be quantitated with the LDL band. This band may not be observed in every specimen. Chylomicrons, if present, remain at the origin.

Upon completion of electrophoresis the lipoprotein bands are stained with enzymic reagent of the present invention, so that the bands' cholesterol content can be quantitated by fluorometric densitometry. The reagent's active ingredients, and the direction of the reaction when the reagent's pH is 7.5-9.5 are shown as follows:

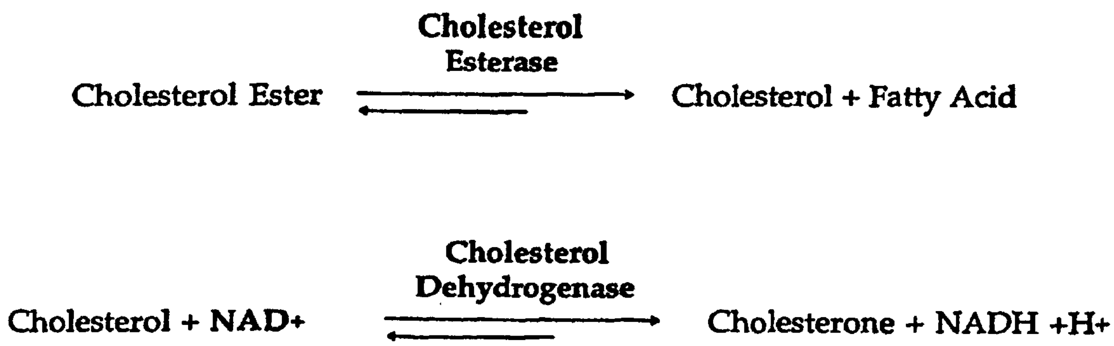


*** The active ingredients of the reagent are shown in bold.**

The amount of NADH produced is directly proportional to the amount of cholesterol and cholesterol esters originally present in the sample. The relative percent cholesterol in each fraction is obtained by scanning in the fluorescent mode using a scanning densitometer, which detects the emissions from excited NADH molecules.

Thus, the invention broadly encompasses a reagent for staining lipoproteins for fluorometric analysis of electrophoretic bands, said reagent comprising enzymic components, and an oxidizing agent component.

The invention further encompasses a method for fluorescent analysis of the cholesterol content of high density lipoproteins (HDL), low density lipoproteins (LDL), and very low density lipoproteins (VLDL) on a electrophoretic plate, the method comprising:

(1) separating the lipoproteins electrophoretically;
(2) staining the separated proteins with a reagent comprising a enzymic component and an oxidizing agent component; and
(3) exciting the stained proteins with an appropriate wavelength of electromagnetic radiation; and
(4) scanning the exited proteins with a densitometer with fluorometric capabilities.

## Detailed Description of the Invention

Any conventional electrophoresis instrument can be used to practice the present invention. In practicing the preferred embodiment described below, Helena Laboratories Corporation's Rapid ElectroPhoresis (REP®) and Rapid ElectroPhoresis 3 (REP® 3) instruments, which are commercially available, have been used. The REP® instrument and the use of this instrument are described in U.S. patent Nos. 4,810,348 and 4,909,920,
The REP® 3 instrument is similar to the REP® instrument, but includes an in situ fluorescent scanner. The REP® 3 instrument and the use of this instrument are described in U.S. patent No. 6,068,753.

The present invention will be explained in the context of a REP® 3 scanning electrophoresis apparatus marketed by Helena Laboratories Corporation.

In the preferred embodiment of the present invention fresh serum samples. are clectrophoresed using an agarose gel matrix or plate. The electrophoresis is carried out under native conditions. In addition to the agarose, the gel used includes quanidine hydrochloride, magnesium chloride or magnesium sulfate, sodium azide and other preservatives and an electrophoresis buffer, preferably sodium barbital with EDTA at a pH range of 7.6 to 9.6. In the preferred embodiment the sodium barbital has a pH of approximately 8.6. Utilizing a Helena Laboratories Corporation Rep® Electrophoresis system, the sample is electrophoresed at 225 volts for 40 minutes at 12°C. It should be understood that other analyzers and systems will likely require different conditions for optimizing the assay.

After the electrophoresis step, the agarose plate is allowed to air dry for approximately 5 minutes. This air dry step is for the purpose of removing excess moisture from the surface of the gel before the reagent is applied. The plate is not completely dried in the this step. The stain or reagent of the present invention is then applied.

The concentration of the reactive ingredients of the reagent is as follows:

| | |
|---|---|
| Cholesterol Esterase (Pseudomnae sp.) | 4.5 U/mL* |
| Cholesterol Dehydrogenase (Nocardia sp.) | 0.9 U/mL* |
| NAD | 29 mM |

*U/mL stands for units per milliliter in the reconstituted reagent (which is discussed below)

NAD is nicotinamide adenine dinucleotide, which is an oxidizing agent. More specifically, NAD is a proton-acceptor co-enzyme. In the reduced form NADH will fluoresce when excited with an appropriate wavelength of electromagnetic radiation.

Prior to use, the reagent's active ingredients are stored apart from the reagent's buffer. To reconstitute the active ingredients and prepare the reagent for use, the active ingredients in the amounts indicated above are dissolved in the inactive ingredient; 885 mM of Tricine Buffer having a pH in the range of 7.5-9.5. The active ingredients must be swirled gently until all of the active ingredients are dissolved. The reconstituted reagent is stable for approximately 2 to 4 hours at 2 to 6°C.

Once the reagent is applied to the electrophoresed plate, it is allowed to incubate with the cholesterol and cholesterol esters for approximately 3 minutes at 30°C. Following the incubation period the electrophoresed plate is dried at 54°C for 3 minutes. Again, it should be understood that other analyzers and systems will likely require different conditions for optimizing the assay. Thereafter, the plate is fluorometrically scanned. Quantitation of the fluorescent patterns has been obtained using the in situ scanner on Helena Laboratories Corporation's REP® 3, but any densitometer with fluorometric capability can be used. The fractions will fluoresce at a peak excitation wavelength of 356nm, due to the presence of NADH, the reduced form of nicotinamide adenine dinucleotide.

## Test Results

A total of 57 patients with total cholesterols* ranging from 124-200 mg/dL were tested using the above identified reagent and procedure with Helena Laboratories Corporation's REP® 3 instrument.[7] Twenty-three (23) of these specimens had Fast pre-beta with normal total cholesterol The following data was obtained:

| N=57 | |
|---|---|
| | RANGE ($\bar{X}$ + 2 SD) |
| HDL (%) | 20.5-49.7 |

*Total Cholesterols were run using a Total Cholesterol method at 37°C.

[7] Lipoprotein cholesterol values vary according to age and sex, and wide variations among different geographical locations and races have been reported. Therefore, it is essential that each laboratory establish its own expected range for its particular population.

(continued)

| N=57 | $\bar{X}$ |
|---|---|
| | RANGE ( + 2 SD) |
| VLDL (%) | 0 - 273 |
| LDL$^+$ pie-beta (%) | 36.6 - 69.4 |

**Results**

The REP® 3 Auto-Flur Cholesterol system separates the major lipoprotein classes. The alpha band which migrates the farthest toward the anode corresponds to HDL. The next band, pre-beta, corresponds to VLDL and the slowest moving beta band corresponds approximately to LDL. If a band appears between alpha and the origin, with fast pre-beta mobility, it corresponds to LDL and should be added to the LDL quantitation. This band may not be observed in every specimen. Chylomicrons, if present, remain at the origin.

**Calculations**

The Helena REP® 3 automatically calculates and prints the relative percent and the absolute values for each band when the specimen total cholesterol is entered.

**Limitations**

This method is intended for the separation and quantitation of lipoprotein classes. For those specimens which exhibit a band between alpha and the origin, with fast pre-beta mobility, it should be reported as part of the total LDL fraction. This band should not be reported as Lp(a) since sufficient clinical studies have not been done to substantiate this decision.

The system is linear to 400 mg/dL total cholesterol, or at least 250 mg/dL per band, with sensitivity to 2 mg/dL per band. Patient sample quantitations which exceed the linearity of the system should be diluted with saline and retested.

**Interpretation of Results**

Treatment decisions of the NCEP guidelines are based primarily on LDL cholesterol levels (Table 1). The risk factors considered in the classification scheme are age (males equal to or older than 45 years and females equal to or older than 55), family history of premature CHD, smoking, hypertension, and diabetes. Treatment is appropriate when LDL cholesterol is at or above the following cut points: all patients at or above 160 mg/dL, with two or more risk factors a value above 130 mg/dL.

HDL cholesterol is considered high risk at or below 35 mg/dL and counted as one of the risk factors in the classification scheme. An HDL cholesterol value above 60 mg/dL is considered protective and subtracts one from the total number of risk factors.

| Treatment Decision Cut-Points | | |
|---|---|---|
| **Total Cholesterol** | | |
| Desirable Blood Cholesterol | <200 mg/dL | |
| Borderline-High Blood Cholesterol | 200-239 mg/dL | |
| High Blood Cholesterol | ≥240 mg/dL | |
| **LDL-Cholesterol** | | |
| **Dietary Therapy** | **Initiation Level** | **LDL Goal** |
| Without CHD and fewer than 2 risk factors | ≥160 mg/dL | <160 mg/dL |
| Without CHD and with 2 or more risk factors | ≥130 mg/dL | <130mg/dL |
| With CHD | >100 mg/dL | ≤100 mg/dL |

(continued)

| Treatment Decision Cut-Points | | |
|---|---|---|
| **LDL Cholesterol** | | |
| **Drug Treatment** | **Initiation Level** | **LDL Goal** |
| Without CHD and fewer than 2 risk factors | ≥190 mg/dL | <160 mg/dL |
| Without CHD and with 2 or more risk factors | ≥160 mg/dL | <130 mg/dL |
| With CHD | ≥130 mg/dL | ≤100 mg/dL |
| HDL-Cholesterol | | |
| Low HDL Cholesterol | <35mg/dL | |
| Protective HDL Cholesterol | >60mg/dL | |
| Triglycerides | | |
| Desirable | <250mg/dL | |
| Borderline | 250-500 mg/dL | |
| Elevated | 500-1000 mg/dL | |
| Severely Elevated/Pancreatitis | >1000 mg/dL | |

**Performance Characteristics**

**Precision**

Within Run

A single patient sample was run a total of thirty times on one gel. This precision study gave CVs of less than 5%. No VLDL was observed in this sample.

| N=30 | | |
|---|---|---|
| | HDL% | LDL% |
| Mean | 25.9 | 74.1 |
| SD | 0.8 | 0.8 |
| CV | 3.0% | 1.0% |

Between Run

Three different patient samples, representing low, middle and high LDL levels, were tested in replica showing excellent reproducibility.

| | | HDL % | | | % VLDL | | | % LDL + fast pre-beta (*if present) | | |
|---|---|---|---|---|---|---|---|---|---|---|
| LDL Concentration (mg/dL) | n | mean | SC | CV | mean | SD | CV | mean | SC | CV |
| ~90 | 80 | 26.2 | 1.6 | 6 | 11.2 | 1.5 | 13.3 | 62.6* | 2.3 | 3.6 |
| ~120 | 80 | 42.6 | 2 | 4.6 | 18 | 1.8 | 10.1 | 39.4 | 2.4 | 6 |
| ~180 | 80 | 37 | 1.8 | 4.8 | 5.8 | 1 | 16.9 | 57.2* | 2.1 | 3.7 |

### Linearity and Sensitivity

Serial dilutions of an elevated cholesterol sample were made and tested by using the methods and reagent of the present invention. The linearity study showed that the system is linear to 400 mg/dL total cholesterol or at least 250 mg/dL per band, and that the system is sensitive to 2.0 mg/dL per band.

### Comparison Studies

Patient samples were run for the correlation study done in conjunction with Helena's REP® 3 Auto-Flur Cholesterol system and the REP® Cholesterol Profile system. The range of total cholesterol for the samples was 115 mg/dL-311 mg/dL. All patients had triglyceride levels below 500 mg/dL. The following is the correlation data for individual bands on the two systems.
X = REP® Cholesterol Profile
Y = REP® 3 Auto Plur Cholesterol

| n = 20 | | |
|---|---|---|
| | Y= | R |
| HDL | 0.804X +5.132 | 0.955 |
| VLDL | 1.361X + 2.335 | 0.932 |
| LDL | 1.166X - 18.772 | 0.884 |
| All Bands | 0.779X + 7.170 | 0.968 |

References to REP® Cholesterol refers to a system where the fractions are visible and reference to REP® 3 Auto Flur Cholesterol refers to laboratory testing of the present invention.[8]

## Claims

1. A method for fluorescent analysis of the cholesterol content of high density lipoproteins (HDL), low density lipoproteins (LDL), and very low density lipoproteins (VLDL) on a electrophoretic plate, said method comprising:

   separating the lipoproteins electrophoretically;
   staining the separated proteins with a reagent comprising at least one enzymic component and an oxidising component,
   exciting the stained proteins with an appropriate wavelength of electromagnetic radiation; and
   fluorometrical scanning the excited proteins with a densitometer with fluorometric capabilities.

2. A method as claimed in claim 1, wherein said method further comprises the step of drying the electrophoretic plate before staining the proteins, and the step of drying the electrophoretic plate after staining the proteins, but before scanning them.

3. A method as claimed in claims 1 or 2, wherein said at least one enzymic component comprises at least two enzymes.

4. A method as claimed in claim 3, wherein one of at least two enzymes is a Cholesterol Dehydrogenase.

5. A method as claimed in claim 4, wherein said Cholesterol Dehydrogenase is isolated from Nocardia sp.

6. A method as claimed in claim 4, wherein a second of said at least two enzymes is a Cholesterol Esterase.

7. A method as claimed in claim 6, wherein the oxidizing agent is a proton acceptor co-enzyme.

8. A method as claimed in claim 7, wherein said reagent further comprises an alkaline buffer.

[8] As of the date the priority application was filed.

**9.** A method as claimed in claim 8, wherein said proton-acceptor co-enzyme is nicotinamide adenine dinucleotide.

**10.** A method as claimed in claim 9, wherein said buffer has a pH of 7.5 to 9.5.

**11.** A method as claimed in claim 10, wherein the buffer is Tricine.

**12.** A method as claimed in any one of claims 4-11, wherein said Cholesterol Dehydrogenase is present in an amount 0.9U/mL.

**13.** A method as claimed in any one of claims 9-12, wherein said NAD is present in an amount 29 mM.

**14.** A method as claimed in any one of claims 6-13, wherein said Cholesterol Esterase is present in an amount 4.5 U/mL.

## Patentansprüche

**1.** Verfahren für die Fluoreszenzanalyse des Cholesteringehaltes von Lipoproteinen hoher Dichte (HDL), Lipoproteinen geringer Dichte (LDL) und Lipoproteinen sehr geringer Dichte (VLDL) auf einer Elektrophoreseplatte, wobei genanntes Verfahren Folgendes umfasst:

Elektrophoretische Trennung der Lipoproteine;
Färben der getrennten Proteine mit einem Reagens, das mindestens eine Enzymkomponente und eine Oxidationskomponente umfasst,
Anregen der gefärbten Proteine mit einer geeigneten Wellenlänge elektromagnetischer Strahlung und
Fluoreszenz-Scanning der angeregten Proteine mit einem Densitometer mit fluorometrischen Fähigkeiten.

**2.** Verfahren nach Anspruch 1, worin genanntes Verfahren ferner den Schritt des Trocknens der Elektrophoreseplatte vor dem Färben der Proteine und den Schritt des Trocknens der Elektrophoreseplatte nach dem Färben der Proteine, aber vor ihrem Scanning umfasst.

**3.** Verfahren nach Anspruch 1 oder 2, worin genannte mindestens eine Enzymkomponente mindestens zwei Enzyme umfasst.

**4.** Verfahren nach Anspruch 3, worin eines von mindestens zwei Enzymen eine Cholesterindehydrogenase ist.

**5.** Verfahren nach Anspruch 4, worin genannte Cholesterindehydrogenase aus Nocardia sp. isoliert wird.

**6.** Verfahren nach Anspruch 4, worin ein zweites von genannten mindestens zwei Enzymen eine Cholesterinesterase ist.

**7.** Verfahren nach Anspruch 6, worin das Oxidationsmittel ein Protonenakzeptor-Coenzym ist.

**8.** Verfahren nach Anspruch 7 worin genanntes Reagens ferner einen alkalischen Puffer umfasst.

**9.** Verfahren nach Anspruch 8, worin genanntes Protonenakzeptor-Coenzym Nicotinamid-adenin-dinucleotid (NAD) ist.

**10.** Verfahren nach Anspruch 9, worin genanter Puffer einen pH von 7,5 bis 9,5 aufweist.

**11.** Verfahren nach Anspruch 10, worin der Puffer Tricin ist.

**12.** Verfahren nach einem der Ansprüche 4 bis 11, worin genannte Cholesterindehydrogenase in einer Menge von 0,9 U/ml vorliegt.

**13.** Verfahren nach einem der Ansprüche 9 bis 12, worin genannte NAD in einer Menge von 29 mM vorliegt.

**14.** Verfahren nach einem der Ansprüche 6 bis 13, worin genannte Cholesterinesterase in einer Menge 4,5 U/ml vorliegt.

## Revendications

1. Méthode pour l'analyse fluorescente de la teneur en cholestérol de lipoprotéines de haute densité (HDL), de lipoprotéines de faible densité (LDL) et de lipoprotéines de très faible densité (VLDL) sur une plaque électrophorétique, ladite méthode comprenant :

    la séparation des lipoprotéines de manière électrophorétique ;
    la coloration des protéines séparées par un réactif comprenant au moins un composant enzymatique et un composant oxydant ;
    l'excitation des protéines colorées par une longueur d'onde appropriée de rayonnement électromagnétique ; et
    le balayage fluorométrique des protéines excitées par un densitomètre ayant des capacités fluorométriques.

2. Méthode selon la revendication 1, dans laquelle ladite méthode comprend en outre l'étape de séchage de la plaque électrophorétique avant la coloration des protéines, et l'étape de séchage de la plaque électrophorétique après la coloration des protéines, mais avant leur balayage.

3. Méthode selon la revendication 1 ou 2, dans laquelle ledit au moins un composant enzymatique comprend au moins deux enzymes.

4. Méthode selon la revendication 3, dans laquelle une parmi au moins deux enzymes est une cholestérol déshydrogénase.

5. Méthode selon la revendication 4, dans laquelle ladite cholestérol déshydrogénase est isolée à partir de Nocardia sp.

6. Méthode selon la revendication 4, dans laquelle une deuxième parmi lesdites au moins deux enzymes est une cholestérol estérase.

7. Méthode selon la revendication 6, dans laquelle l'agent oxydant est une coenzyme accepteuse de protons.

8. Méthode selon la revendication 7, dans laquelle ledit réactif comprend en outre un tampon alcalin.

9. Méthode selon la revendication 8, dans laquelle ladite coenzyme accepteuse de protons est le nicotinamide adénine dinucléotide.

10. Méthode selon la revendication 9, dans laquelle ledit tampon a un pH allant de 7,5 à 9,5.

11. Méthode selon la revendication 10, dans laquelle le tampon est la Tricine.

12. Méthode selon l'une quelconque des revendications 4 à 11, dans laquelle la cholestérol déshydrogénase est présente en une quantité de 0,9 U/mL.

13. Méthode selon l'une quelconque des revendications 9 à 12, dans laquelle ledit NAD est présent en une quantité de 29 mM.

14. Méthode selon l'une quelconque des revendications 6 à 13, dans laquelle la cholestérol estérase est présente en une quantité de 4,5 U/mL.